(19) Europäisches Patentamt / European Patent Office / Office européen des brevets

(11) **EP 4 379 448 B1**

(12) **EUROPEAN PATENT SPECIFICATION**

(45) Date of publication and mention
of the grant of the patent:
**22.04.2026 Bulletin 2026/17**

(21) Application number: **22848547.0**

(22) Date of filing: **26.07.2022**

(51) International Patent Classification (IPC):
$G01N\ 21/64^{(2006.01)}$   $A61B\ 5/00^{(2006.01)}$
$G02B\ 26/10^{(2006.01)}$

(52) Cooperative Patent Classification (CPC):
**G01N 21/6456; A61B 5/0071; G02B 26/105;**
G01N 2021/6463; G01N 2201/1053; G02B 26/101

(86) International application number:
**PCT/CN2022/108039**

(87) International publication number:
**WO 2023/005942 (02.02.2023 Gazette 2023/05)**

(54) **LASER SCANNING IMAGING METHOD AND SYSTEM, STORAGE MEDIUM, AND COMPUTER PROGRAM**

LASERABTASTBILDGEBUNGSVERFAHREN UND -SYSTEM, SPEICHERMEDIUM UND COMPUTERPROGRAMM

PROCÉDÉ ET SYSTÈME D'IMAGERIE PAR BALAYAGE LASER, SUPPORT DE STOCKAGE ET PROGRAMME INFORMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.07.2021 CN 202110852227**

(43) Date of publication of application:
**05.06.2024 Bulletin 2024/23**

(73) Proprietors:
• **Wuxi Hisky Medical Technologies Co., Ltd.**
  **Taihu International Science & Technology Park**
  **Xinwu District, Wuxi**
  **Jiangsu 214000 (CN)**
• **Suzhou Microview Medical Technologies Co., Ltd.**
  **Suzhou, Jiangsu 215000 (CN)**

(72) Inventors:
• **SUN, Shibo**
  **Wuxi, Jiangsu 214000 (CN)**
• **HE, Qiong**
  **Wuxi, Jiangsu 214000 (CN)**
• **SHAO, Jinhua**
  **Wuxi, Jiangsu 214000 (CN)**
• **SUN, Jin**
  **Suzhou, Jiangsu 215000 (CN)**

(74) Representative: **Elzaburu S.L.P.**
  **Edificio Torre de Cristal**
  **Paseo de la Castellana 259 C, planta 28**
  **28046 Madrid (ES)**

(56) References cited:
CN-A- 113 558 576    CN-A- 113 558 577
JP-A- 2001 013 944   JP-A- 2003 344 781
JP-A- 2015 025 900   JP-A- 2018 081 136
JP-A- 2020 173 428   JP-A- H11 271 621
US-A1- 2003 063 367  US-A1- 2006 157 638
US-A1- 2006 157 638

EP 4 379 448 B1

**Description**

**TECHNICAL FIELD**

The present invention relates to laser scanning imaging technologies and, in particular, to a laser scanning optical imaging method, a system, a storage medium and a computer program.

**BACKGROUND**

Cancer related cases are increasing in modern lifestyle. A laser scanning imaging system has a very bright prospect in the application of early detection, screening and diagnosis of cancer due to its ability to provide in real time tissue information at subcellular resolution. The laser scanning imaging system includes main parts such as a laser, a laser scanning device, an objective lens, a pinhole, and an optical sensor.

In the laser scanning imaging process, the laser of the laser scanning imaging system emits a laser beam. The laser scanning device deflects the laser beam for scanning, the objective lens focuses the laser beam on the biological tissues, the irradiated biological tissues generate fluorescence information, and the fluorescence information passes through the objective lens, the laser scanning device and the pinhole to reach the optical sensor and is received by the optical sensor, which forms the fluorescence information of the irradiated area.

A resonant galvanometer is used as a fast mirror in the laser scanning device and usually performs a nonlinear motion. Along with the motion of the resonant galvanometer, the laser scanning device outputs a galvanometer scanning synchronization signal, which is a square wave representing a change in the direction of the motion of the galvanometer. A hardware clock board is typically used to receive the galvanometer scanning synchronization signal and generate a clock signal for sampling the fluorescence signal.

Currently, the clock signal is generated by the hardware clock board, in this way, the clock signal is generated only in a fixed portion of the motion of the resonant galvanometer that is relatively linear, so that the corresponding imaging range of the resonant galvanometer is relatively small and the imaging position is fixed, and the characteristics of the clock signal generated in this way are fixed and cannot be flexibly altered with the change of the scanning range, which results in a relatively small and fixed area of the fluorescence image. Even if the imaging area is subsequently changed by means of amplification processing and the like, the actual resolution of the fluorescence image still can't be increased.

US 20060157638A1 discloses a scanning microscope which includes a light source unit that projects laser light, an optical system that converges and applies the laser light onto a data obtaining point on a specimen, a data obtaining order deciding unit that decides a data obtaining order in which adjacent data obtaining points are not consecutive, a scanning unit that scans the laser light in accordance with the data obtaining order, a detector that detects detection light from the data obtaining point, a storage unit that stores luminance information on the detection light detected by the detector in association with positional information on the data obtaining point, and an image formation unit that forms a two-dimensional image on the basis of the association of the luminance information with the positional information.

JP 2003344781A discloses a scanning means (5) which reflects or refracts the light from a light source while vibrating it to irradiate a sample with the light, a means (23) for obtaining speed information on the scanning means, a means (60) which absorbs temporal delay from the irradiation of the sample with the scanning light to the generation of a sampling clock, a means (24) for correcting the amplitude with a quantity associated with the scanning state of the scanning means, a means (25) which makes the speed information an absolute value to generate absolute speed information, a means (27) which adjusts the absolute value speed information to a specified amplitude, means (28, 61) of correcting levels, a means (29) of generating the sampling clock of frequency corresponding to the corrected absolute value speed information, and a means (30) of managing the number of sampling clocks corresponding to the scanning of the scanning means and an imaging period of the sample.

JP 2020173428A discloses that a sampling circuit comprising a PLL unit 21 that creates, with a phase lock group, an N multiplication clock that is N times the scanning frequency of a scanning position signal of a resonant scanner and is synchronous with the phase of the scanning position signal; an AD converter 23 that synchronizes a detection signal obtained by detecting light from a sample with the N multiplication clock to perform AD conversion; a counter unit 25 that counts the number of clocks in synchronization with the N multiplication clock; a counter threshold storage memory 27 that holds a counter threshold corresponding to a desired scanning position of the resonant scanner; a comparison unit 29 that, when the counter threshold matches a counter value of the counter unit 25, outputs a sampling clock; and a data processing unit 31 that samples the AD-converted detection signal based on the sampling clock to create pixel data.

**SUMMARY**

In view of the above problem existing in the prior art, the present invention provides a laser scanning imaging method, a system, a storage medium and a computer program, which can realize that the generated clock signal can be flexibly altered with the change in the range of the effective area, so as to present a user with a scanning imaging result that meets requirements. The present invention is set out in the appended set of claims.

An embodiment of the present application provides a laser scanning imaging method as defined in appended independent claim 1.

An embodiment of the present application also provides a laser scanning imaging system as defined in appended independent claim 7.

An embodiment of the present application further provides a storage medium as defined in appended claim 8.

An embodiment of the present application further provides a computer program as defined in appended claim 9.

Compared with the prior art, the present application generates a clock signal based on the received galvanometer scanning synchronization signal and parameter information related to the effective area, samples a fluorescence signal received from the galvanometer scanning according to the clock signal, obtains the fluorescence image information of the effective area, and realizes that the generated clock signal can be flexibly altered with the changes in the range of the effective area, so as to obtain the fluorescence image of the effective area, and presents the user with the scanning imaging result that satisfies the requirement.

**BRIEF DESCRIPTION OF DRAWINGS**

In the accompanying drawings, which are not necessarily drawn to scale, the same reference signs may depict similar components in different figures. The same reference signs with letter suffixes or different letter suffixes may represent different instances of similar components. The drawings illustrate various embodiments generally by way of example rather than limitation, and are used in conjunction with the description and the claims to illustrate the disclosed embodiments. Where appropriate, the same reference signs are used in all of the drawings to refer to the same or similar parts. Such embodiments are illustrative and are not intended to be exhaustive or exclusive embodiments of the present device or method.

FIG. 1 is a flowchart of a laser scanning imaging method according to an embodiment of the present application.
FIG. 2 is another flowchart of a laser scanning imaging method according to an embodiment of the present application.
FIG. 3 is yet another flowchart of a laser scanning imaging method according to an embodiment of the present application.
FIG. 4 is still another flowchart of a laser scanning imaging method according to an embodiment of the present application.
FIG. 5 is a schematic diagram of a hardware structure of an electronic device provided by an embodiment of the present application.

**DESCRIPTION OF EMBODIMENTS**

With reference to the accompanying drawings, various embodiments as well as features of the present application are described.

The accompanying drawings, which are included in and form a part of the specification, illustrate embodiments of the present application and are used to explain the principles of the application in conjunction with the general description of the application given above and the detailed description of the embodiments given below.

These and other features of the present application will become apparent by the following description of alternative forms of the embodiments given as non-limiting examples with reference to the accompanying drawings.

It should also be understood that although the present application has been described with reference to some specific embodiments, within the scope defined by the appended claims, many other equivalent forms of the present application can be realized with certainty by those skilled in the art.

The aforementioned and other aspects, features and advantages of the present application will become more apparent in view of the following detailed description when taken in conjunction with the accompanying drawings.

Specific embodiments of the present application are described with reference to the accompanying drawings. However, it should be understood that the embodiments are merely examples of the present application, which may be implemented in a variety of ways, within the scope defined by the appended claims. Well-known and/or repeated functions and structures are not described in detail to avoid unnecessary or redundant details that would lead to ambiguity of the present application. Accordingly, the specific structural and functional details invented herein are not intended to be limiting, but are merely intended to serve as a basis for the claims and as a representative basis for teaching those skilled in the art to substantially utilize the application in a variety of ways with any suitable detailed structure.

This specification may use the phrases "in one embodiment", "in another embodiment", "in yet another embodiment", or "in other embodiments", which refer to one or more of the same or different embodiments according to the present application.

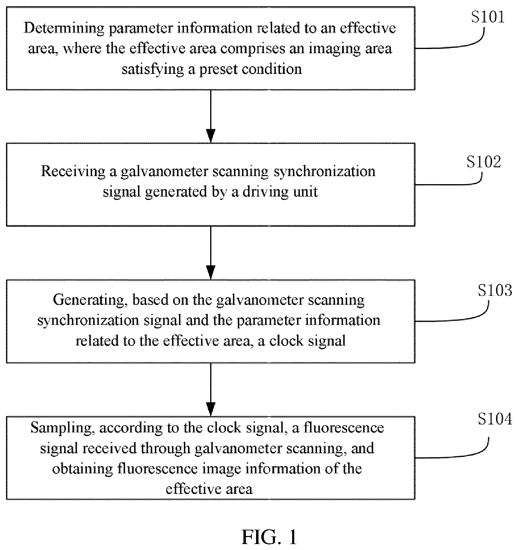

Embodiments of the present application provide a laser scanning imaging method that can be applied to a laser scanning imaging system. As shown in FIG. 1, the laser scanning imaging method includes steps S101 to S104.

Step S101: determining parameter information related to an effective area, where the effective area comprises an imaging area satisfying a preset condition.

Step S102: receiving a galvanometer scanning synchronization signal generated by a driving unit.

Step S103: generating, based on the galvanometer scanning synchronization signal and the parameter information related to the effective area, a clock signal.

Step S104: sampling, according to the clock signal, a fluorescence signal received through galvanometer scanning, and obtaining fluorescence image information of the effective area.

Specifically, the galvanometer may be a linear galvanometer, a nonlinear galvanometer, or a composition of both. If the galvanometer is a linear galvanometer or a nonlinear galvanometer, the one-dimensional fluorescence image information of the effective area is obtained by the steps S101 to S104; if the galvanometer includes a linear galvanometer and a nonlinear galvanometer, the two-dimensional fluorescence image information of the effective area is obtained by the steps S101 to S104. The nonlinear galvanometer is the resonant galvanometer mentioned before.

Specifically, an imaging area that satisfies a preset condition, for example an area that needs fluorescence imaging, may be set by a user. The effective area can be understood as the imaging area that needs to be displayed for the user. It should be noted that the effective area can be the entire scanning area of the laser scanning imaging system, or a part of the entire scanning area, depending on the user's requirement.

Specifically, the driving unit may be a nonlinear driving unit. The nonlinear galvanometer in the laser scanning imaging system performs natural oscillation and nonlinear motion after powered up. The nonlinear driving unit generates a galvanometer scanning synchronization signal of nonlinear galvanometer, and the galvanometer scanning synchronization signal synchronizes with the motion of the nonlinear galvanometer, where the specific expression of the galvanometer scanning synchronization signal may be a square wave signal. The laser scanning imaging system receives the galvanometer scanning synchronization signal and generates a clock signal based on the galvanometer scanning synchronization signal and the parameter information related to the effective area. In this way, the generated clock signal can be flexibly changed with the changes in the range of the effective area of imaging, which can meet the user's imaging needs for various areas and resolutions and avoid the problem in the prior art that the hardware clock board can only generate a fixed clock signal for fluorescence signal sampling through receiving the galvanometer scanning synchronization signal, resulting in only a fixed area for imaging.

Through the solution of the present application, the user can set the imaging area (i.e., the effective area) according to the imaging requirements, and it can be realized that the generated clock signal can be flexibly altered with the changes in the range of the effective area, and then the sampling is performed based on the clock signal to obtain the fluorescence image of the effective area, so as to present the user with a scanning imaging result that satisfies the requirements.

In some embodiments, the parameter information includes area range information of the effective area.

In some embodiments, the area range information at least includes a starting position, an end position, and the number of pixel points.

Specifically, for the imaging area is displayed for the user according to the requirements, a starting position, an end position and the number of pixel points of the effective area may be determined, where the imaging area displayed for the user is the area with an imaging requirement of the user. The above mentioned starting position, the end position and the number of pixels are parameter information related to the effective area, where the starting position and the end position may be expressed by means of coordinates (one-dimensional, two-dimensional or three-dimensional coordinates) or a deflection angle of the galvanometer.

Alternatively, for example, when the user needs to zoom in on a result of the scanning imaging, the zoomed in result of the scanning imaging is displayed by increasing the number of pixels in the effective area without changing the preset values of the starting position and the end position, or by shortening the distance between the starting position and the end position without changing the number of pixels, thus realizing that the displayed result of the scanning imaging is zoomed in. Alternatively, for example, when the user needs to zoom out on the result of the scanning imaging, the zoomed out result of the scanning imaging is displayed by decreasing the number of pixels in the effective area without changing the preset values of the starting position and the end position, or by increasing the distance between the starting position and the end position without changing the number of pixels, thus realizing that the displayed result of the scanning imaging is zoomed out. The number of pixels refers to the number of pixel points.

A nonlinear galvanometer is taken as an example of the galvanometer in the following.

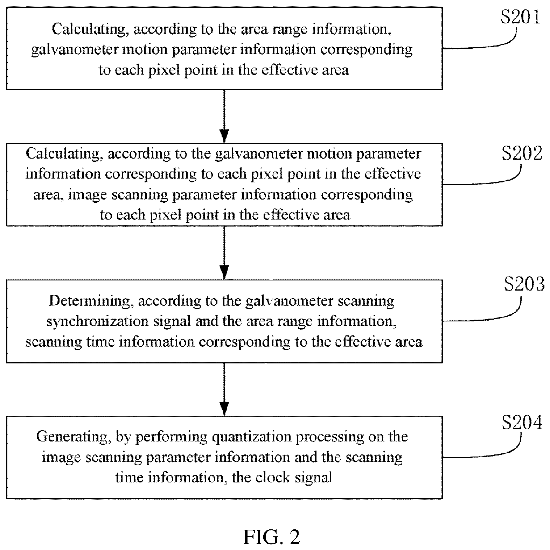

As shown in FIG. 2, the Step S103: generating, based on the galvanometer scanning synchronization signal and the parameter information related to the effective area, a clock signal, specifically includes steps S201 to S204.

Step S201: calculating, according to the area range information, galvanometer motion parameter information corresponding to each pixel point in the effective area.

Step S202: calculating, according to the galvanometer motion parameter information corresponding to each pixel point in the effective area, image scanning parameter information corresponding to each pixel point in the effective area.

Step S203: determining, according to the galvanometer scanning synchronization signal and the area range information, scanning time information corresponding to the effective area.

Step S204: generating, by performing quantization processing on the image scanning parameter information and the scanning time information, the clock signal.

In some embodiments, the galvanometer motion parameter information includes at least one of the followings: motion speed information of the galvanometer, motion acceleration information of the galvanometer, motion frequency information of the galvanometer, deflection angle information of the galvanometer, spatial position information at an edge of the galvanometer, and spatial position information of a mirror of the galvanometer. The motion frequency information of the galvanometer can also be understood as the swing frequency information of the galvanometer.

In some embodiments, the image scanning parameter information includes at least one of the followings: sampling position information corresponding to each of the pixel points, sampling moment information corresponding to each of the pixel points, and sampling time information corresponding to each of the pixel points.

In some embodiments, the scanning time information includes at least any two scanning moments corresponding to the effective area. Alternatively, when the scanning time information includes any two scanning moments corresponding to the effective area, the any two scanning moments corresponding to the effective area may be a starting moment and an end moment, a starting moment and any one scanning moment other than the starting moment, or an end moment and any one scanning moment other than the end moment.

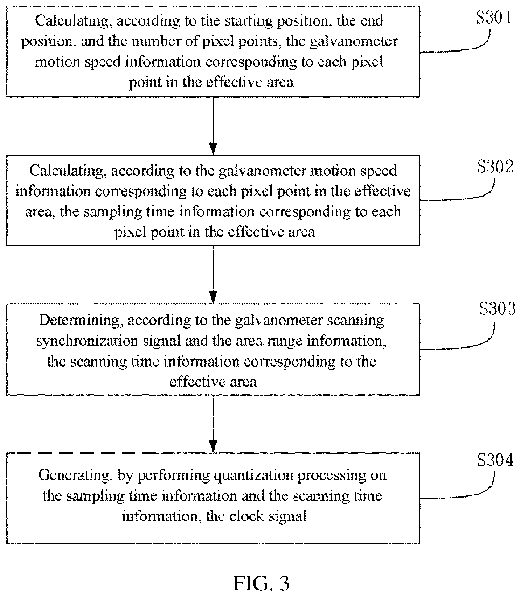

The area range information at least includes the starting position, the end position, and the number of pixel points. The following takes the galvanometer motion parameter information including the motion speed information of the galvanometer, and the image scanning parameter information including the sampling time information corresponding to each pixel point, as an example, as shown in FIG. 3, and presents a specific scheme of generating a clock signal, including the following steps S301 to S304:

Step 301: calculating, according to the starting position, the end position, and the number of pixel points, the galvanometer motion speed information corresponding to each pixel point in the effective area.

Step 302: calculating, according to the galvanometer motion speed information corresponding to each pixel point in the effective area, the sampling time information corresponding to each pixel point in the effective area.

Step 303: determining, according to the galvanometer scanning synchronization signal and the area range information, the scanning time information corresponding to the effective area.

Specifically, in this embodiment, the scanning time information includes the starting moment and the end moment.

Step 304: generating, by performing quantization processing on the sampling time information and the scanning time information, the clock signal; that is, generating, by performing quantization processing on the sampling time information, the starting moment and the end moment, the clock signal.

Specifically, when the starting position and the end position are expressed in the form of one-dimensional coordinates, the corresponding coordinate values can be transformed into the corresponding deflection angle $\theta$ of the nonlinear galvanometer by the formula $x = d*\tan\theta$, where d is a galvanometer distance parameter, such as a perpendicular distance from the center of the nonlinear galvanometer to the scanning plane, and x is the coordinate value of the current pixel point. Then, the galvanometer motion speed information corresponding to each pixel point can be obtained by taking the derivative of the galvanometer deflection angle $\theta$ corresponding to each pixel point.

Specifically, when the starting position and the end position are expressed in the form of the deflection angle $\theta$ of the galvanometer, the motion speed of the nonlinear galvanometer corresponding to each pixel point in the effective area may be calculated according to a motion function that may be a trigonometric function describing simple harmonic oscillation. For a more accurate description, the function may be obtained by measurement, and the parameters used for the calculation in the motion function at least includes the maximum motion position of the nonlinear galvanometer (e.g., the maximum deflection angle), the motion frequency of the nonlinear galvanometer (which is reciprocal relationship to the oscillation period of the nonlinear galvanometer), where the deflection angle of the galvanometer can also be understood as the motion angle of the galvanometer, then the maximum deflection angle of the galvanometer can be the maximum motion angle of the galvanometer.

Alternatively, it can be concluded from the above mentioned that the motion speed of the nonlinear galvanometer corresponding to each pixel point in the effective area is $V(P_n)$, where V is the speed, P is the pixel point, n ranges from 1 to N, and N is the number of pixel points in the effective area. Then taking the reciprocal of the motion speed of the nonlinear galvanometer corresponding to each pixel point, the sampling time informa-tion $\dfrac{1}{V(P_n)}$ corresponding to each pixel point can be obtained. Next, taking the sampling time information corresponding to the pixel point P(m) with the largest speed as a standard, the sampling time information corresponding to each pixel point is quantized to obtain the quantized sampling time information corresponding to each pixel point; then by performing quantization processing on the above quantized sampling time information, the starting moment and the end moment, a clock signal corresponding to the effective area is generated.

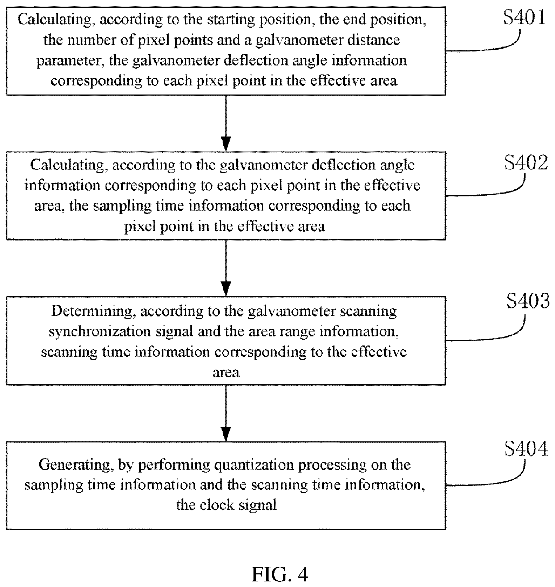

The area range information at least includes a starting position, an end position, and the number of pixel points. The following takes the galvanometer motion parameter information including the galvanometer deflection angle information, and the image scanning parameter information including the sampling time information corresponding to each pixel point, as an example, as shown in FIG. 4, and introduces another specific scheme for generating a clock signal, including the following steps S401 to S404:

Step 401: calculating, according to the starting position, the end position, the number of pixel points and a galvanometer distance parameter, the galvanometer deflection angle information corresponding to each pixel point in the effective area.

Step 402: calculating, according to the galvanometer deflection angle information corresponding to each pixel point in the effective area, the sampling time information corresponding to each pixel point in the effective area.

Step 403: determining, according to the galvanometer scanning synchronization signal and the area range information, scanning time information corresponding to the effective area.

Specifically, in this embodiment, the scanning time information includes a starting moment and an end moment.

Step 404: generating, by performing quantization processing on the sampling time information and the scanning time information, the clock signal. That is, generating, by performing quantization processing on the sampling time information, the starting moment and the end moment, the clock signal.

Specifically, according to the starting position, the end position and the number of pixel points, a relative coordinate of each pixel point in the effective area relative to the starting position is determined as $x(P_n)$, where x is the relative coordinate, P is a pixel point, n ranges from 1 to N, and N is the number of pixel points in the effective area. By means of the galvanometer distance parameter, such as the perpendicular distance from the center of the nonlinear galvanometer to the scanning plane, the deflection angel information of the nonlinear galvanometer corresponding to each pixel point is determined as $\theta(P_n)$. According to the deflection angle information $\theta(P_n)$ of the nonlinear galvanometer corresponding to each pixel point, the sampling time information $T(P_n)$ corresponding to each pixel point can be obtained by utilizing the description formula of the deflection angle of the nonlinear galvanometer. Then by performing quantization processing on the sampling time information, the starting moment and the end moment, the clock signal associated with the effective area can be generated.

The above specific scheme of generating the clock signal is illustrative and not limiting. The scheme of generating the clock signal by the galvanometer scanning synchronization signal and the parameter information related to effective area is within the protection scope of this invention as defined by the appended claims.

In some embodiments, the area range information at least includes the starting position and the end position, where the step S203: determining, based on the galvanometer scanning synchronization signal and the area range information, scanning time information corresponding to the effective area includes:

determining, according to the starting position and a period of the galvanometer scanning synchronization signal, a starting moment corresponding to the effective area;

determining, according to the end position and a period of the galvanometer scanning synchronization signal, an end moment corresponding to the effective area.

Specifically, the following formula can be used to calculate the starting moment and end moment corresponding to the effective area: $\theta=\theta_{max}*P(f*t)$, where P is a function describing the change of the angle of the nonlinear galvanometer over time, and this function can be a trigonometric function describing the simple harmonic oscillation; f is the motion frequency of the nonlinear galvanometer, which can also be called as the swing frequency of the nonlinear galvanometer and can be determined based on the period of the galvanometer scanning synchronization signal; and $\theta_{max}$ is the maximum deflection angle (i.e., the maximum motion angle) of the nonlinear galvanometer. The above formula describes the one-to-one correspondence between the motion angle of the nonlinear galvanometer and time. Therefore, the starting moment corresponding to the effective area can be calculated from the starting position, and the end moment corresponding to the effective area can be calculated from the end position.

In some embodiments, the galvanometer includes a first galvanometer and a second galvanometer, where the first galvanometer and the second galvanometer are set vertically, and the laser scanning method further includes:

determining, according to the galvanometer scanning synchronization signal and the effective area, a control signal for the second galvanometer;

controlling and driving the first galvanometer to move in a first direction;

controlling, according to the control signal of the second galvanometer, the second galvanometer to move in a second direction.

Specifically, the driving unit may be a nonlinear driving unit. The first galvanometer may be the nonlinear galvanometer as previously described, and the second galvanometer may be the linear galvanometer as previously described. The first direction may be a direction perpendicular to the second direction, e.g., the first direction is an X-axis direction in the rectangular coordinate system, and the second direction is a Y-axis direction in the rectangular coordinate system, that is, the above-described non-linear galvanometer can move in the X-axis direction, and the above-described linear galvanometer can move in the Y-axis direction. Alternatively, the X-axis direction can be a horizontal direction and the Y-axis direction can be a vertical direction.

Taking the first direction being the horizontal direction, the second direction being the vertical direction, and the effective area being a part of the entire scanning area as an example, the following explains the process of obtaining fluorescence image information in the present embodiment: from a scanning starting position of the entire scanning area, the nonlinear galvanometer is first driven to move along the horizontal direction of the scanning starting position, and when the nonlinear galvanometer is moved to a maximum scanning position along the horizontal direction, the linear galvanometer is driven to move along the vertical direction. When the linear galvanometer moves to the next position along the vertical direction, the nonlinear galvanometer is driven to move along the horizontal direction. The above motion process is repeated until the first galvanometer and the second galvanometer cooperate to complete the motion in the entire scanning area. During the above motion process of the two galvanometers, the fluorescence signal in the effective area is sampled according to the generated clock signal related to the effective area, and the two-dimensional fluorescence image information of the effective area can be obtained.

By the scheme of the present embodiment, the two-dimensional fluorescence image information can ultimately be obtained by the two galvanometers moving in two mutually perpendicular directions respectively, which further improves the accuracy of the obtained fluorescence image information, obtains image information with more dimensions, and provides a guarantee of the accuracy of the subsequent diagnosis.

In some embodiments, the determining, based on the galvanometer scanning synchronization signal and the effective area, a control signal for the second galvanometer includes: determining, based on the galvanometer scanning synchronization signal and the parameter information related to the effective area, the control signal for the second galvanometer.

Specifically, the parameter information may include a starting position, an end position, and the number of pixel points, these parameters are related to the effective area, and based on these information, the motion of the linear galvanometer can be determined, thereby realizing the control of the motion thereof, so as to ultimately obtain a fluorescence image of the effective area.

Embodiments of the present application also provide a laser scanning imaging system including a galvanometer and a processor connected to the galvanometer, where the processor is used to execute the laser scanning imaging method provided in any one of the above embodiments.

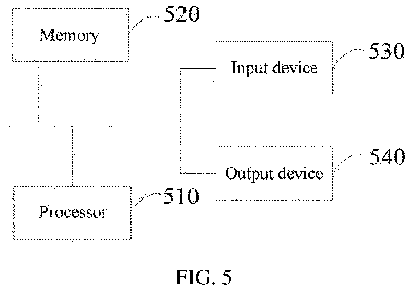

FIG. 5 is a schematic diagram of a hardware structure of a laser scanning imaging system for performing the laser scanning imaging method provided by an embodiment of the present application, and as shown in FIG. 5, the laser scanning imaging system includes: one or more processors 510 and a memory 520. Taking the processor 510 in FIG. 5 as an example: the laser scanning imaging system for performing the laser scanning imaging method may also include: an input device 530 (including a galvanometer) and an output device 540.

The processor 510, the memory 520, the input device 530 and the output device 540 may be connected via a bus or otherwise. In FIG. 5, a connection via a bus is used as an example.

The memory 520 serves as a non-volatile computer-readable storage medium that can be used to store a non-volatile software program, a non-volatile computer-executable program and a module, such as the program instructions/module corresponding to the laser scanning imaging method in embodiments of the present application. The processor 510 executes various functional applications and data processing of the server by running the non-volatile software program, instructions, and module stored in the memory 520, that is, realizing the laser scanning imaging method in the above embodiments.

The memory 520 may include a high-speed random access memory, and may also include a non-volatile memory, such as at least one disk memory device, a flash memory device, or other non-volatile solid state memory device. Example of the aforementioned network includes, but not limited to, the Internet, an enterprise intranet, a local area network, a mobile communication network, and a combination thereof.

With the schemes of the present application, the user can set the effective area according to the imaging requirement, and realize that the generated clock signal can be flexibly altered with the change of the range of the effective area, so as to perform the sampling based on of the clock signal to obtain the fluorescence image within the effective area, presenting the user with the scanning imaging result that satisfies the requirement.

Embodiments of the present application also provide a non-volatile storage medium storing a computer program, which is executed by a processor to realize the steps of the laser scanning imaging method according to any one embodiment of the present application. A person of ordinary skill in the art may understand that realizing all or some of the steps carried by the method of the above embodiments can be accomplished by a program to instruct the associated hardware, and the program may be stored in a medium, and when the program is executed, one of the steps of the method embodiments or a combination thereof is realized. The medium may be a storage medium or a signal medium. The storage medium may be an electronic, magnetic, optical, electromagnetic, infrared, or semiconductor system, apparatus or device, or any combination of the foregoing, but is not limited thereto; specifically, it may be: a portable computer disk, a hard disk, a random access memory (RAM), a read-only memory (ROM), an erasable programmable read-only memory (EPROM) (or flash memory), an optical fiber, a portable compact disc read-only memory (CD-ROM), an optical storage device, a magnetic storage device, or any combination of the foregoing. The signal medium may include, but is not limited to, an electrical signal medium, an optical signal medium, a radio wave (electromagnetic wave) medium, or any combination of the foregoing.

In addition, the various functional units in various embodiments of the present application may be integrated in a single processing module, or the individual unit may physically exist separately, or two or more units may be integrated in a single module. The integrated modules may be implemented either in the form of hardware or in the form of software functional modules. The integrated module may also be stored in a computer-readable storage medium if it is realized in the form of a software function module and sold or used as a separate product.

The above mentioned storage medium may be a read-only memory, a disk or a CD-ROM, etc.

It should be noted that the various units according to various embodiments of the present application may be realized as computer-executable instructions stored on a memory which, when executed by a processor, can realize the corresponding steps; or as hardware with corresponding logical computational capabilities; or as a combination of software and hardware (firmware). In some embodiments, the processor may be implemented as any one of a field programmable gate array (FPGA), an application specific integrated circuit (ASIC), a digital signal processor (DSP) chip, System on Chip (SOC), a microprocessor unit (MPU) (for example but not limited to, the Cortex®), and the like.

It should be noted that in the various components of the system of the present application, the components are logically divided according to the functions to be realized thereof, however, the present application is not limited to that. Within the scope defined by the claims, the various components may be re-divided or combined as desired, for example, some components may be combined into a single component, or some components may be optically broken down into more sub-components.

The various component embodiments of the present application may be implemented in hardware, or in software modules running on one or more processors, or in combination thereof. Those skilled in the art should understand that microprocessors or digital signal processors (DSPs) may be used in practice to implement some or all of the functionality of some or all of the components of a system according to embodiments of the present application. The present application may also be implemented as an apparatus or device program (e.g., computer program and computer program product) for performing some or all of the methods described herein. The program implementing the present application may be stored on a computer-readable medium or may have the form of one or more signals. Such signals may be available through downloading from an Internet site, or provided on a carrier signal or in any other form. In addition, the present application may be realized with by means of hardware including a number of different elements and by means of a suitably programmed computer. In unit claims enumerating a number of devices, several of these devices may be embodied by means of the same hardware item. The use of the words first, second and third, etc. does not indicate any order. The words may be construed as names.

Furthermore, although exemplary embodiments have been described herein, the scope thereof includes any and all embodiments having an equivalent element, modification, omission, combination (e.g., schemes in which various embodiments intersect), adaptation, or alteration based on the present application. The elements in the claims will be construed broadly based on the language employed in the claims and are not limited to the examples described in this specification or during the implementation of this application, and the embodiments will be construed as non-exclusive. Accordingly, this specification and the embodiments are intended to be considered merely exemplary.

The above description is intended to be illustrative and not limiting. For example, the above examples (or one or more schemes thereof) may be used in combination with one another. For example, a person of ordinary skill in the art may use other embodiments when reading the above description. Also, in the specific embodiments described above, various features may be grouped together to simplify the present application. This should not be construed as an intent that features of the disclosure that do not require protection are necessary for any claim. Rather, the subject matter of the present application may be less than all of the features of a particularly disclosed embodiment. Thereby, the following claims are incorporated here as examples or embodiments in specific implementation mode, where each claim independently serves as a separate embodiment, and it is contemplated that these embodiments may be combined with each other in various combinations or permutations, as defined by the claims and their specified dependencies.

The above embodiments are only exemplary embodiments of the present application and are not intended to limit the present application. The protection scope of the present application is limited by the claims.

**Claims**

1. A laser scanning imaging method, comprising:

   determining (S101) parameter information related to an effective area, wherein the effective area comprises an imaging area satisfying a preset condition;

receiving (S102) a galvanometer scanning synchronization signal generated by a driving unit, wherein the galvanometer scanning synchronization signal is a square wave representing a change in the direction of the motion of the galvanometer;

generating (S103), based on the galvanometer scanning synchronization signal and the parameter information related to the effective area, a clock signal; and

sampling (S104), according to the clock signal, a fluorescence signal received through galvanometer scanning, and obtaining fluorescence image information of the effective area;

wherein the parameter information comprises area range information of the effective area;

wherein the generating, based on the galvanometer scanning synchronization signal and the parameter information related to the effective area, a clock signal comprises:

> calculating (S201), according to the area range information, galvanometer motion parameter information corresponding to each pixel point in the effective area;
>
> calculating (S202), according to the galvanometer motion parameter information corresponding to each pixel point in the effective area, image scanning parameter information corresponding to each pixel point in the effective area;
>
> determining (S203), according to the galvanometer scanning synchronization signal and the area range information, scanning time information corresponding to the effective area; and
>
> generating (S204), by performing quantization processing on the image scanning parameter information and the scanning time information, the clock signal;
>
> wherein the area range information at least comprises a starting position, an end position, and the number of pixel points;
>
> **characterized in that**,
>
> > i) the calculating, according to the area range information, galvanometer motion parameter information corresponding to each pixel point in the effective area comprises: calculating (S301), according to the starting position, the end position, and the number of pixel points, the galvanometer motion speed information corresponding to each pixel point in the effective area; and
> >
> > the calculating, according to the galvanometer motion parameter information corresponding to each pixel point in the effective area, image scanning parameter information corresponding to each pixel point in the effective area comprises: calculating (S302), according to a reciprocal of the galvanometer motion speed information corresponding to each pixel point in the effective area, sampling time information corresponding to each pixel point in the effective area; or

ii) the calculating, according to the area range information, galvanometer motion parameter information corresponding to each pixel point in the effective area comprises: calculating (S401), according to the starting position, the end position, the number of pixel points, and a galvanometer distance parameter, galvanometer deflection angle information corresponding to each pixel point in the effective area; and

the calculating, according to the galvanometer motion parameter information corresponding to each pixel point in the effective area, image scanning parameter information corresponding to each pixel point in the effective area comprises: calculating (S402), according to the galvanometer deflection angle information corresponding to each pixel point in the effective area, sampling time information corresponding to each pixel point in the effective area.

2.  The laser scanning imaging method according to claim 1, wherein the galvanometer motion parameter information comprises at least one of the followings: motion speed information of the galvanometer, motion acceleration information of the galvanometer, deflection angle information of the galvanometer, motion frequency information of the galvanometer, spatial position information at an edge of the galvanometer, and spatial position information of a mirror of the galvanometer.

3.  The laser scanning imaging method according to claim 1 or claim 2, wherein the image scanning parameter information comprises at least one of the followings: sampling position information corresponding to the each pixel point, sampling moment information corresponding to the each pixel point, and sampling time information corresponding to the each pixel point.

4.  The laser scanning imaging method according to claim 1, wherein the determining, according to the galvanometer scanning synchronization signal and the area range information, scanning time information corresponding to the effective area comprises:

> determining, according to the starting position and a period of the galvanometer scanning synchronization signal, a starting moment corresponding to the effective area;
> determining, according to the end position and a period of the galvanometer scanning synchronization signal, an end moment corresponding to the effective area.

5. The laser scanning imaging method according to claim 1, wherein the galvanometer comprises a first galvanometer and a second galvanometer, the first galvanometer and the second galvanometer are set vertically, and the method further comprises:

> determining, according to the galvanometer scanning synchronization signal and the effective area, a control signal for the second galvanometer;
> controlling the driving unit to drive the first galvanometer to move in a first direction;
> controlling, according to the control signal of the second galvanometer, the second galvanometer to move in a second direction.

6. The laser scanning imaging method according to claim 5, wherein the determining, according to the galvanometer scanning synchronization signal and the effective area, a control signal for the second galvanometer comprises:
determining, according to the galvanometer scanning synchronization signal and the parameter information related to the effective area, the control signal for the second galvanometer.

7. A laser scanning imaging system, **characterized by** comprising:

> a galvanometer (530);
> a driving unit configured for generating a galvanometer scanning synchronization signal; and
> a processor (510), connected to the galvanometer (530), for executing the laser scanning imaging method according to any one of claims 1 to 6.

8. A storage medium (520), storing a computer program, wherein the computer program, when executed by the processor (510) of the system according to claim 7, causes the system according to claim 7 to execute the laser scanning imaging method according to any one of claims 1 to 6.

9. A computer program, wherein the computer program, when executed by the processor (510) of the system according to claim 7, causes the system according to claim 7 to execute the laser scanning imaging method according to any one of claims 1 to 6.

## Patentansprüche

1. Laserabtastbildgebungsverfahren, umfassend:

> Bestimmen (S101) von Parameterinformationen, die sich auf einen effektiven Bereich beziehen, wobei der effektive Bereich einen Bildgebungsbereich umfasst, der eine vorgegebene Bedingung erfüllt;
> Empfangen (S102) eines Galvanometer-Abtastsynchronisationssignals, das durch eine Antriebseinheit erzeugt wird, wobei es sich bei dem Galvanometer-Abtastsynchronisationssignal um eine Rechteckwelle handelt, die eine Änderung in der Bewegungsrichtung des Galvanometers darstellt;
> Erzeugen (S103) eines Taktsignals basierend auf dem Galvanometer-Abtastsynchronisationssignal und den Parameterinformationen, die sich auf den effektiven Bereich beziehen; und
> Abtasten (S104) eines durch Galvanometerabtastung empfangenen Fluoreszenzsignals gemäß dem Taktsignal, und Erlangen von Fluoreszenzbildinformationen des effektiven Bereichs;
> wobei die Parameterinformationen Bereichsreichweiteninformationen des effektiven Bereichs umfassen;
> wobei das Erzeugen eines Taktsignals basierend auf dem Galvanometer-Abtastsynchronisationssignal und den Parameterinformationen, die sich auf den effektiven Bereich beziehen, Folgendes umfasst:

>> Berechnen (S201) von Galvanometer-Bewegungsparameterinformationen, die jedem Pixelpunkt in dem effektiven Bereich entsprechen, gemäß den Bereichsreichweiteninformationen;
>> Berechnen (S202) von Bildabtastparameterinformationen, die jedem Pixelpunkt in dem effektiven Bereich entsprechen, gemäß den Galvanometer-Bewegungsparameterinformationen, die jedem Pixelpunkt in dem effektiven Bereich entsprechen;
>> Bestimmen (S203) von Abtastzeitinformationen, die dem effektiven Bereich entsprechen, gemäß dem Galvanometer-Abtastsynchronisationssignal und den Bereichsreichweiteninformationen; und

Erzeugen (S204) des Taktsignals durch Durchführen von Quantisierungsverarbeitung an den Bildabtastparameterinformationen und den Abtastzeitinformationen;

wobei die Bereichsreichweiteninformationen mindestens eine Startposition, eine Endposition und die Anzahl von Pixelpunkten umfassen;

**dadurch gekennzeichnet, dass**

i) das Berechnen von Galvanometer-Bewegungsparameterinformationen, die jedem Pixelpunkt in dem effektiven Bereich entsprechen, gemäß den Bereichsreichweiteninformationen Folgendes umfasst: Berechnen (S301) von Galvanometer-Bewegungsgeschwindigkeitsinformationen, die jedem Pixelpunkt in dem effektiven Bereich entsprechen, gemäß der Startposition, der Endposition und der Anzahl von Pixelpunkten; und

das Berechnen von Bildabtastparameterinformationen, die jedem Pixelpunkt in dem effektiven Bereich entsprechen, gemäß den Galvanometer-Bewegungsparameterinformationen, die jedem Pixelpunkt in dem effektiven Bereich entsprechen, Folgendes umfasst: Berechnen (S302) von Abtastzeitinformationen, die jedem Pixelpunkt in dem effektiven Bereich entsprechen, gemäß einem Kehrwert der Galvanometer-Bewegungsgeschwindigkeitsinformationen; oder

ii) das Berechnen von Galvanometer-Bewegungsparameterinformationen, die jedem Pixelpunkt in dem effektiven Bereich entsprechen, gemäß den Bereichsreichweiteninformationen Folgendes umfasst: Berechnen (S401) von Galvanometer-Ablenkwinkelinformationen, die jedem Pixelpunkt in dem effektiven Bereich entsprechen, gemäß der Startposition, der Endposition, der Anzahl von Pixelpunkten und einem Galvanometer-Abstandsparameter; und

das Berechnen von Bildabtastparameterinformationen, die jedem Pixelpunkt in dem effektiven Bereich entsprechen, gemäß den Galvanometer-Bewegungsparameterinformationen, die jedem Pixelpunkt in dem effektiven Bereich entsprechen, Folgendes umfasst: Berechnen (S402) von Abtastzeitinformationen, die jedem Pixelpunkt in dem effektiven Bereich entsprechen, gemäß den Galvanometer-Ablenkwinkelinformationen, die jedem Pixelpunkt in dem effektiven Bereich entsprechen.

2. Laserabtastbildgebungsverfahren nach Anspruch 1, wobei die Galvanometer-Bewegungsparameterinformationen mindestens eines von Folgendem umfassen: Bewegungsgeschwindigkeitsinformationen des Galvanometers, Bewegungsbeschleunigungsinformationen des Galvanometers, Ablenkwinkelinformationen des Galvanometers, Bewegungsfrequenzinformationen des Galvanometers, räumliche Positionsinformationen an einer Kante des Galvanometers und räumliche Positionsinformationen eines Spiegels des Galvanometers.

3. Laserabtastbildgebungsverfahren nach Anspruch 1 oder Anspruch 2, wobei die Bildabtastparameterinformationen mindestens eines von Folgendem umfassen: Abtastpositionsinformationen, die jedem Pixelpunkt entsprechen, Abtastzeitpunktinformationen, die jedem Pixelpunkt entsprechen, und Abtastzeitinformationen, die jedem Pixelpunkt entsprechen.

4. Laserabtastbildgebungsverfahren nach Anspruch 1, wobei das Bestimmen von Abtastzeitinformationen, die dem effektiven Bereich entsprechen, gemäß dem Galvanometer-Abtastsynchronisationssignal und den Bereichsreichweiteninformationen Folgendes umfasst:

Bestimmen eines Startzeitpunkts, der dem effektiven Bereich entspricht, gemäß der Startposition und einer Zeitspanne des Galvanometer-Synchronisationssignals;

Bestimmen eines Endzeitpunkts, der dem effektiven Bereich entspricht, gemäß der Endposition und einer Zeitspanne des Galvanometer-Synchronisationssignals.

5. Laserabtastbildgebungsverfahren nach Anspruch 1, wobei das Galvanometer ein erstes Galvanometer und ein zweites Galvanometer umfasst, das erste Galvanometer und das zweite Galvanometer vertikal eingestellt sind, und das Verfahren ferner Folgendes umfasst:

Bestimmen eines Steuersignals für das zweite Galvanometer gemäß dem Galvanometer-Synchronisationssignal und dem effektiven Bereich;

Steuern der Antriebseinheit, um das erste Galvanometer in eine erste Richtung zu bewegen;

Steuern des zweiten Galvanometers, um es gemäß dem Steuersignal des zweiten Galvanometers in eine zweite Richtung zu bewegen.

**6.** Laserabtastbildgebungsverfahren nach Anspruch 5, wobei das Bestimmen eines Steuersignals für das zweite Galvanometer gemäß dem Galvanometer-Synchronisationssignal und dem effektiven Bereich Folgendes umfasst:

Bestimmen des Steuersignals für das zweite Galvanometer gemäß dem Galvanometer-Synchronisationssignal und den Parameterinformationen, die sich auf den effektiven Bereich beziehen.

**7.** Laserabtastbildgebungssystem, **dadurch gekennzeichnet, dass** es Folgendes umfasst:

ein Galvanometer (530);
eine Antriebseinheit, die konfiguriert ist, um ein Galvanometer-Synchronisationssignal zu erzeugen; und
einen Prozessor (510), der mit dem Galvanometer (530) verbunden ist, um das Laserabtastbildgebungsverfahren nach einem der Ansprüche 1 bis 6 auszuführen.

**8.** Speichermedium (520), das ein Computerprogramm speichert, wobei das Computerprogramm, wenn es durch den Prozessor (510) des Systems nach Anspruch 7 ausgeführt wird, das System nach Anspruch 7 veranlasst, das Laserabtastbildgebungsverfahren nach einem der Ansprüche 1 bis 6 auszuführen.

**9.** Computerprogramm, wobei das Computerprogramm, wenn es durch den Prozessor (510) des Systems nach Anspruch 7 ausgeführt wird, das System nach Anspruch 7 veranlasst, das Laserabtastbildgebungsverfahren nach einem der Ansprüche 1 bis 6 auszuführen.

**Revendications**

**1.** Procédé d'imagerie par balayage laser, comprenant :

la détermination (S101) d'informations de paramètres relatives à une zone effective, dans lequel la zone effective comprend une zone d'imagerie satisfaisant une condition prédéfinie ;
la réception (S102) d'un signal de synchronisation de balayage de galvanomètre généré par une unité d'entraînement, dans lequel le signal de synchronisation de balayage de galvanomètre est une onde carrée représentant un changement dans la direction du mouvement du galvanomètre ;
la génération (S103), sur la base du signal de synchronisation de balayage de galvanomètre et des informations de paramètres relatives à la zone effective, d'un signal d'horloge ; et
l'échantillonnage (S104), selon le signal d'horloge, d'un signal de fluorescence reçu par balayage de galvanomètre, et l'obtention d'informations d'image de fluorescence de la zone effective ;
dans lequel les informations de paramètres comprennent des informations de plage de zones de la zone effective ;
dans lequel la génération, sur la base du signal de synchronisation de balayage de galvanomètre et des informations de paramètres relatives à la zone effective, d'un signal d'horloge comprend :

le calcul (S201), selon les informations de plage de zones, d'informations de paramètres de mouvement de galvanomètre correspondant à chaque point de pixel dans la zone effective ;
le calcul (S202), selon les informations de paramètres de mouvement de galvanomètre correspondant à chaque point de pixel dans la zone effective, d'informations de paramètres de balayage d'image correspondant à chaque point de pixel dans la zone effective ;
la détermination (S203), selon le signal de synchronisation de balayage de galvanomètre et les informations de plage de zones, d'informations de temps de balayage correspondant à la zone effective ; et
la génération (S204), en réalisant un traitement de quantification sur les informations de paramètres de balayage d'image et les informations de temps de balayage, du signal d'horloge ;
dans lequel les informations de plage de zones comprennent au moins une position de départ, une position d'arrivée et le nombre de points de pixels ;
**caractérisé en ce que**,

i) le calcul, selon les informations de plage de zones, d'informations de paramètres de mouvement de galvanomètre correspondant à chaque point de pixel dans la zone effective comprend :
le calcul (S301), selon la position de départ, la position d'arrivée et le nombre de points de pixel, des informations de vitesse de mouvement de galvanomètre correspondant à chaque point de pixel dans la zone effective ; et
le calcul, selon les informations de paramètres de mouvement de galvanomètre correspondant à chaque point de pixel dans la zone effective, d'informations de paramètres de balayage d'image correspondant à chaque point de pixel dans la zone effective comprend : le calcul (S302), selon l'inverse des informations de vitesse de mouvement de galvanomètre correspondant à chaque point pixel dans la zone effective, d'informations de temps d'échantillonnage correspondant à chaque point pixel dans la zone effective ; ou ii) le calcul, selon les informations de plage de zones, d'informations de paramètres de mouvement de galvanomètre correspondant à chaque point de pixel dans la zone effective comprend : le calcul (S401), selon la position de départ, la position d'arrivée, le nombre de points de pixel et un paramètre de distance de galvanomètre, d'informations d'angle de déviation de galvanomètre correspondant à chaque point de pixel dans la zone effective ; et

le calcul, selon les informations de paramètres de mouvement de galvanomètre correspondant à chaque point de pixel dans la zone effective, d'informations de paramètres de balayage d'image correspondant à chaque point de pixel dans la zone effective comprend : le calcul (S402), selon les informations d'angle de déviation de galvanomètre correspondant à chaque point de pixel dans la zone effective, d'informations de temps d'échantillonnage correspondant à chaque point de pixel dans la zone effective.

2. Procédé d'imagerie par balayage laser selon la revendication 1, dans lequel les informations de paramètres de mouvement de galvanomètre comprennent au moins l'un des éléments suivants : informations de vitesse de mouvement du galvanomètre, informations d'accélération de mouvement du galvanomètre, informations d'angle de déviation du galvanomètre, informations de fréquence de mouvement du galvanomètre, informations de position spatiale sur un bord du galvanomètre et informations de position spatiale d'un miroir du galvanomètre.

3. Procédé d'imagerie par balayage laser selon la revendication 1 ou la revendication 2, dans lequel les informations de paramètres de balayage d'image comprennent au moins l'un des éléments suivants : informations de position d'échantillonnage correspondant à chaque point de pixel, informations de moment d'échantillonnage correspondant à chaque point de pixel et informations de temps d'échantillonnage correspondant à chaque point de pixel.

4. Procédé d'imagerie par balayage laser selon la revendication 1, dans lequel la détermination, selon le signal de synchronisation de balayage de galvanomètre et les informations de plage de zones, d'informations de temps de balayage correspondant à la zone effective comprend :

la détermination, selon la position de départ et une période du signal de synchronisation de balayage de galvanomètre, d'un moment de départ correspondant à la zone effective ; la détermination, selon la position d'arrivée et une période du signal de synchronisation de balayage de galvanomètre, d'un moment d'arrivée correspondant à la zone effective.

5. Procédé d'imagerie par balayage laser selon la revendication 1, dans lequel le galvanomètre comprend un premier galvanomètre et un second galvanomètre, le premier galvanomètre et le second galvanomètre sont disposés verticalement, et le procédé comprend en outre :

la détermination, selon le signal de synchronisation de balayage de galvanomètre et la zone effective, d'un signal de commande pour le second galvanomètre ; la commande de l'unité d'entraînement pour entraîner le premier galvanomètre à se déplacer dans une première direction ; la commande, selon le signal de commande du second galvanomètre, du second galvanomètre à se déplacer dans une seconde direction.

6. Procédé d'imagerie par balayage laser selon la revendication 5, dans lequel la détermination, selon le signal de synchronisation de balayage de galvanomètre et la zone effective, d'un signal de commande pour le second galvanomètre comprend : la détermination, selon le signal de synchronisation de balayage de galvanomètre et les informations de paramètres relatives à la zone effective, du signal de commande pour le second galvanomètre.

7. Système d'imagerie par balayage laser, caractérisé en comprenant :

un galvanomètre (530) ; une unité d'entraînement configurée pour générer un signal de synchronisation de balayage de galvanomètre ; et un processeur (510), connecté au galvanomètre (530), pour exécuter le procédé d'imagerie par balayage laser selon l'une quelconque des revendications 1 à 6.

8. Support de stockage (520), stockant un programme informatique, dans lequel le programme informatique, lorsqu'il est exécuté par le processeur (510) du système selon la revendication 7, amène le système selon la revendication 7 à exécuter le procédé d'imagerie par balayage laser selon l'une quelconque des revendications 1 à 6.

9. Programme informatique, dans lequel le programme informatique, lorsqu'il est exécuté par le processeur (510) du système selon la revendication 7, amène le système selon la revendication 7 à exécuter le procédé d'imagerie par balayage laser selon l'une quelconque des revendications 1 à 6.

Determining parameter information related to an effective area, where the effective area comprises an imaging area satisfying a preset condition

S101

Receiving a galvanometer scanning synchronization signal generated by a driving unit

S102

Generating, based on the galvanometer scanning synchronization signal and the parameter information related to the effective area, a clock signal

S103

Sampling, according to the clock signal, a fluorescence signal received through galvanometer scanning, and obtaining fluorescence image information of the effective area

S104

FIG. 1

Calculating, according to the area range information, galvanometer motion parameter information corresponding to each pixel point in the effective area

S201

Calculating, according to the galvanometer motion parameter information corresponding to each pixel point in the effective area, image scanning parameter information corresponding to each pixel point in the effective area

S202

Determining, according to the galvanometer scanning synchronization signal and the area range information, scanning time information corresponding to the effective area

S203

Generating, by performing quantization processing on the image scanning parameter information and the scanning time information, the clock signal

S204

FIG. 2

```
┌─────────────────────────────────────────────┐
│ Calculating, according to the starting       │          S301
│ position, the end position, and the number   │
│ of pixel points, the galvanometer motion     │
│ speed information corresponding to each pixel │
│ point in the effective area                   │
└─────────────────────────────────────────────┘
                       │
                       ▼
┌─────────────────────────────────────────────┐
│ Calculating, according to the galvanometer    │          S302
│ motion speed information corresponding to each │
│ pixel point in the effective area, the         │
│ sampling time information corresponding to     │
│ each pixel point in the effective area         │
└─────────────────────────────────────────────┘
                       │
                       ▼
┌─────────────────────────────────────────────┐
│ Determining, according to the galvanometer    │          S303
│ scanning synchronization signal and the area   │
│ range information, the scanning time           │
│ information corresponding to the effective     │
│ area                                           │
└─────────────────────────────────────────────┘
                       │
                       ▼
┌─────────────────────────────────────────────┐
│ Generating, by performing quantization         │          S304
│ processing on the sampling time information    │
│ and the scanning time information, the clock   │
│ signal                                         │
└─────────────────────────────────────────────┘
```

FIG. 3

Calculating, according to the starting position, the end position, the number of pixel points and a galvanometer distance parameter, the galvanometer deflection angle information corresponding to each pixel point in the effective area

S401

Calculating, according to the galvanometer deflection angle information corresponding to each pixel point in the effective area, the sampling time information corresponding to each pixel point in the effective area

S402

Determining, according to the galvanometer scanning synchronization signal and the area range information, scanning time information corresponding to the effective area

S403

Generating, by performing quantization processing on the sampling time information and the scanning time information, the clock signal

S404

FIG. 4

FIG. 5

# EP 4 379 448 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060157638 A1
- JP 2003344781 A
- JP 2020173428 A